# EUROPEAN PATENT SPECIFICATION

(11) **EP 1 651 221 B1**
(45) Date of publication and mention of the grant of the patent: **14.01.2009**
(21) Application number: 04741115.2
(22) Date of filing: 17.07.2004
(51) Int. Cl.: A61K 31/46, A61K 31/167, A61K 31/137, A61K 31/00, A61P 11/06, A61K 9/00

(54) **MEDICAMENTS FOR INHALATION COMPRISING BETAMIMETICS AND AN ANTICHOLINERGIC AGENT**
MEDIKAMENTE ZUR INHALATION MIT BETAMIMETIKA UND EINEM ANTICHOLINERGIKUM
MEDICAMENTS POUR INHALATION COMPORTANT DES AGENTS A ACTIVITE BETAMIMETIQUE ET ANTICHOLINERGIQUE

(30) Priority: 28.07.2003 EP 03017036
(43) Date of publication of application: 03.05.2006
(73) Proprietor: BOEHRINGER INGELHEIM INTERNATIONAL GMBH, D-55216 Ingelheim (DE); Boehringer Ingelheim Pharma GmbH & Co. KG, 55216 Ingelheim (DE)
(72) Inventor: GERMEYER, Sabine, Sandy Hook, CT 06842 (US); MEADE, Christopher, John, Montague, 88437 Maselheim (DE); MEISSNER, Helmut, 55218 Ingelheim (DE); MORSCHHAEUSER, Gerd, 88400 Biberach (DE); PAIRET, Michel, 88400 Biberach (DE); PESTEL, Sabine, 88448 Attenweiler (DE); PIEPER, Michael, P., 88400 Biberach (DE); POHL, Gerald, 88400 Biberach (DE); REICHL, Richard, 55435 Gau-Algesheim (DE); SPECK, Georg, 55218 Ingelheim (DE); KONETZKI, Ingo, 88447 Warthausen (DE)
(74) Representative: Hammann, Heinz
(86) International application number: PCT/EP2004/007997
(87) International publication number: WO 2005/013992

(56) References cited:
- WO-A-03/000241
- WO-A-03/064419
- DE-A- 10 050 994
- DE-A- 10 050 995
- CALVO G M ET AL: "IS IT USEFUL TO ADD AN ANTICHOLINERGIC TREATMENT TO BETA2-ADRENERGIC MEDICATION IN ACUTE ASTHMA ATTACK" JOURNAL OF INVESTIGATIONAL ALLERGOLOGY AND CLINICAL IMMUNOLOGY, BARCELONA, ES, vol. 8, no. 1, January 1998 (1998-01), pages 30-34, XP009018610 ISSN: 1018-9068
- NOORD VAN J A ET AL: "LONG-TERM TREATMENT OF CHRONIC OBSTRUCTIVE PULMONARY DISEASE WITH SALMETEROL AND THE ADDITIVE EFFECT OF IPRATROPIUM" EUROPEAN RESPIRATORY JOURNAL, MUNKSGAARD INTERNATIONAL PUBLISHERS, COPENHAGEN, DK, vol. 15, no. 5, May 2000 (2000-05), pages 878-885, XP001021107 ISSN: 0903-1936
- WESSELING G ET AL: "COMPARISON OF THE EFFECTS OF ANTICHOLINERGIC AND BETA2-AGONIST AND COMBINATION THERAPY ON RESPIRATORY IMPEDANCE IN COPD" CHEST, THE COLLEGE, CHICAGO, IL, US, vol. 101, no. 1, 1992, pages 166-173, XP000913861 ISSN: 0012-3692

## Description

The present invention relates to novel propellant-free inhalable solutions or suspensions containing beta₂ agonists and salts of a new anticholinergic, processes for preparing them and their use in the treatment of respiratory complaints.

### Description of the invention

The present invention relates to novel propellant-free inhalable solutions or suspensions containing beta₂ agonists and salts of a new anticholinergic **1** processes for preparing them and their use in the treatment of respiratory complaints.

Within the scope of the present invention the anticholinergic agents used are the salts of formula 1 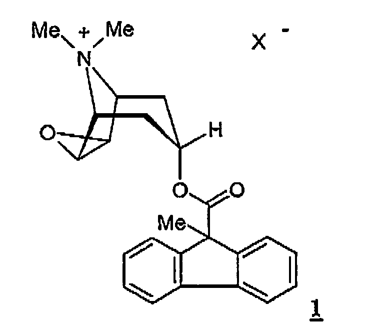 wherein
- X⁻: denotes an anion with a single negative charge, preferably an anion selected from the group consisting of fluoride, chloride, bromide, iodide, sulphate, phosphate, methanesulphonate, nitrate, maleate, acetate, citrate, fumarate, tartrate, oxalate, succinate, benzoate and p-toluenesulphonate,
with the proviso that a solution (in mg/100 ml) consisting of:

| | |
|---|---|
| a compound of formula **1** | 333,3 mg /100 ml, |
| formoterol fumarate | 333,3 mg/100 ml, |
| benzalkoniumchloride | 10,0 mg/100 ml, |
| EDTA | 50,0 mg/100 ml and |
| HCl (1n) | ad pH 3.4 |

is excluded.

Preferably, the salts of formula **1** are used wherein
- X⁻: denotes an anion with a single negative charge selected from among the fluoride, chloride, bromide, 4-toluenesulphonate and methanesulphonate, preferably bromide.

Most preferably, the salts of formula **1** are used wherein
- X⁻: denotes an anion with a single negative charge selected from among the chloride, bromide and methanesulphonate, preferably bromide.

Particularly preferred according to the invention is the salt of formula **1** wherein X⁻ denotes bromide.

Surprisingly, an unexpectedly beneficial therapeutic effect can be observed in the treatment of inflammatory and/or obstructive diseases of the respiratory tract if the anticholinergic of formula **1** is used with one or more betamimetics **2**.

This effect may be observed both when the two active substances are administered simultaneously in a single active substance formulation and when they are administered successively in separate formulations. According to the invention, it is preferable to administer the two active substance ingredients simultaneously in a single formulation. Within the scope of the present invention, any reference to the compound **1**' is to be regarded as a reference to the pharmacologically active cation of the following formula contained in the salts **1** : 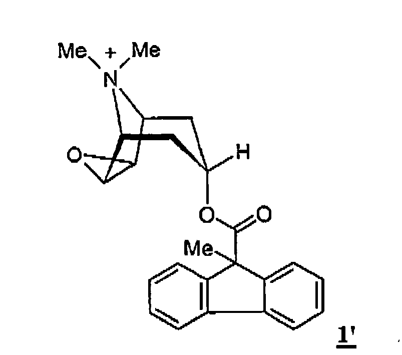 with the proviso that a solution (in mg/100 ml) consisting of :

| | |
|---|---|
| a compound salt of formula **1'** | 333,3 mg /100 ml, |
| formoterol fumarate | 333,3 mg/100 ml, |
| benzalkoniumchloride | 10,0 mg/100 ml, |
| EDTA | 50,0 mg/100 ml and |
| HCl (1n) | ad pH 3.4 |

is excluded.

In the pharmaceutical combinations mentioned above the active substances may be combined in a single preparation or contained in two separate formulations. Pharmaceutical compositions which contain the active substances **1** and **2** in a single preparation are preferred according to the invention.

According to the instant invention preferred beta₂ agonists **2** in the combinations according to the invention are selected from the group consisting of albuterol, bambuterol, bitolterol, broxaterol, carbuterol, clenbuterol, fenoterol, formoterol, hexoprenaline, ibuterol, isoetharine, isoprenaline, levosalbutamol, mabuterol, meluadrine, metaproterenol orciprenaline, pirbuterol, procaterol, reproterol, rimiterol, ritodrine, salmeterol, salmefamol, soterenot, sulphonterol, tiaramide, terbutaline, tolubuterol, CHF-1035, HOKU-81, KUL-1248,3-(4-{6-[2-Hydroxy-2-(4-hydroxy-3-hydroxymethyl-phenyl)-ethylamino]-hexyloxy}-butyl)-benzenesulfoneamide, 5-[2-(5,6-Diethyl-indan-2-ylamino)-1-hydroxy-ethyl]-8-hydroxy-1*H*-quinolin-2-one, 4-hydroxy-7-[2-{[2-{[3-(2-phenylethoxy)propyl]sulphonyl}ethyl]-amino}ethyl]-2(3H)-benzothiazolone, 1-(2-fluoro-4-hydroxyphenyl)-2-[4-(1-benzimidazolyl)-2-methyl-2-butylamino]ethanol, 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-one, 1-(4-amino-3-chloro-5-trifluormethylphenyl)-2-tert.-butylamino)ethanol and 1-(4-ethoxycarbonylamino-3-cyano-5-fluorophenyl)-2-(tert.-butylamino)ethanol, optionally in the form of the racemates, the enantiomers, the diastereomers and optionally the pharmacologically acceptable acid addition salts and the hydrates thereof.

According to the instant invention more preferred beta₂ agonists 2 are selected from the group consisting of bambuterol, bitolterol, carbuterol, clenbuterol, fenoterol, formoterol, hexoprenaline, ibuterol, pirbuterol, procaterol, reproterol, salmeterol, sulphonterol, terbutaline, tolubuterol, 3-(4-{6-[2-Hydrozy-2-(4-hydroxy-3-hydroxymethyl-phenyl)-ethylamino]-hexyloxy}-butyl)-benzenesulfoneamide, 5-[2-(5,6-Diethyl-indan-2-ylamino)-1-hydroxy-ethyl]-8-hydroxy-1*H*-quinolin-2-one, 4-hydroxy-7-[2-{[2-{[3-(2-phenylethoxy)propyl]sulphonyl}ethyl]-amino}ethyl]-2(3H)-benzothiazolone, 1-(2-fluoro-4-hydroxyphenyl)-2-[4-(1-benzimidaxolyl)-2-methyl-2-butylamino]ethanol, 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylammophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triaxol-3-yl]-2-methyl-2-butylamino}ethanol, 5-hydroxy-8-(1-hydroxy-2-isopropylaminobutyl)-2H-1,4-benzoxazin-3-(4H)-one, 1-(4-amino-3-chloro-5-trifluormethylphenyl)-2-tert.-butylamino)ethanol and 1-(4-ethoxycarbonylamino-3-cyano-5-fluorophenyl)-2-(tert.-butylamino)ethanol, optionally in the form of the racemates, the enantiomers, the diastereomers and optionally the pharmacologically acceptable acid addition salts and the hydrates thereof,

More preferably, the betamimetics **2** used as within the compositions according to the invention are selected from among fenoterol, formoterol, salmeterol, 3-(4-{6-[2-Hydroxy-2-(4-hydroxy-3-hydroxymethyl-phenyl)-ethylamino]-hexyloxy}-butyl)-benzenesulfoneamide, 5-[2-(5,6-Diethyl-indan-2-ylamino)-1-hydroxy-ethyl]-8-hydroxy-1*H*-quinolin-2-one, 1-[3-{4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H- 5-hydroxy-3-oxo-4H-1,4-benzoxazin-8-yl]-2- {4-[3-(4-methoxyphenyl)-1,2,4-triazol-3-yl]-2-methyl-2-butylamino}ethanol, optionally in the form of the racemates, the enantiomers, the diastereomers and optionally the pharmacologically acceptable acid addition salts thereof, and the hydrates thereof. Of the betamimetics mentioned above the compounds formoterol, salmeterol, 3-(4-{6-[2-Hydroxy-2-(4-hydroxy-3-hydroxymethyl-phenyl)-ethylamino]-hexyloxy}-butyl)-benzenesulfoneamide, and 5-[2-(5,6-Diethyl-indan-2-ylamino)-1-hydroxy-ethyl]-8-hydrox)-1*H*-quinolin-2-one are particularly preferred, optionally in the form of the racemates, the enantiomers, the diastereomers and optionally the pharmacologically acceptable acid addition salts thereof, and the hydrates thereof. Of the betamimetics mentioned above the compounds formoterol and salmeterol are particularly preferred, optionally in the form of the racemates, the enantiomers, the diastereomers and optionally the pharmacologically acceptable acid addition salts thereof, and the hydrates thereof.

Examples of pharmacologically acceptable acid addition salts of the betamimetics **2** according to the invention are the pharmaceutically acceptable salts which are selected from among the salts of hydrochloric acid, hydrobromic acid, sulphuric acid, phosphoric acid, methanesulphonic acid, acetic acid, fumaric acid, succinic acid, lactic acid, citric acid, tartaric acid, 1-hydroxy 2-naphthalenecarboxylic acid, 4-phenylcinnamic acid, 5-(2.4-difluorophenyl)salicylic acid or maleic acid. If desired, mixtures of the abovementioned acids may also be used to prepare the salts **2**.

According to the invention, the salts of the betamimetics **2** selected from among the hydrochloride, hydrobromide, sulphate, phosphate, fumarate, methanesulphonate, 4-phenylcinnamate, 5-(2.4-difluorophenyl)salicylate, maleate and xinafoate are preferred.

Particularly preferred are the salts of **2** in the case of salmeterol selected from among the hydrochloride, sulphate, 4-phenylcinnamate, 5-(2.4-difluorophenyl)salicylate and xinafoate, of which the 4-phenylcinnamate, 5-(2.4-difluorophenyl)salicylate and especially xinafoate are particularly important Particularly preferred are the salts of **2** in the case of formoterol selected from the hydrochloride, sulphate and fumarate, of which the hydrochloride, and fumarate are particularly preferred. Of exceptional importance according to the invention is formoterol fumarate.

Salts of salmeterol, formoterol, 3-(4-{6-[2-hydroxy-2-(4-hydroxy-3-hydroxymethylphenyl)-ethylamino]-hexyloxy}-butyl)-benzenesulfoneamide, and 5-(2-(5,6-Dietlayl-indan-2-ylamino)-1-hydroxy-ethyl]-8-hydroxy-1*H*-quinolin-2-one, are preferably used as the betamimetics **2** according to the invention. Of particular importance according to the invention are salmeterol and formoterol, salts. Any reference to the term betamimetics **2** also includes a reference to the relevant enantiomers or mixtures thereof.

In the pharmaceutical compositions according to the invention, the compounds **2** may be present in the form of their racemates, enantiomers or mixtures thereof. The separation of the enantiomers from the racemates may be carried out using methods known in the art (e.g. by chromatography on chiral phases, etc.) If the compounds **2** are used in the form of their enantiomers, it is particularly preferable to use the enantiomers in the R configuration at the C-OH group.

As an example, any reference to the most preferred compounds **2** according to the invention, the salts of salmeterol and formoterol, also includes the relevant enantiomeric salts of *R*-salmeterol, *S*-salmeterol, *R,R*-formoterol, *S,S*-formoterol, *R,S*-formoterol, *S,R-*formoterol and the mixtures thereof, while the enantiomeric salts of *R*-salmeterol and *R,R-*formoterol are of particular importance. The compounds **2** may also be present according to the invention in the form of the hydrates or solvates thereof.

Where the present invention refers to betamimetics which are not in the form of salts, this is indicated by a reference to compounds **2'**. For example, the preferred betamimetics **2'** according to the invention which are not in salt form include the free base of formoterol, salmeterol whereas the particularly preferred compounds **2** according to the invention are salmeterol xinafoate or formoterol fumarate.

Within the scope of the present invention the betamimetics **2** may possibly also be referred to as sympathomimetics or beta-₂-agonists (β₂-agonists). All these terms are to be regarded as interchangeable for the purposes of the present invention.

In one aspect the present invention relates to the abovementioned pharmaceutical compositions which contain, in addition to therapeutically effective quantities of **1** and **2** a pharmaceutically acceptable carrier. In another aspect the present invention relates to the abovementioned pharmaceutical compositions which do not contain any pharmaceutically acceptable carrier in addition to therapeutically effective quantities of **1** and **2**.
The present invention also relates to the use of therapeutically effective quantities of the salts **1** for preparing a pharmaceutical composition containing betamimetics 2 for treating inflammatory or obstructive diseases of the respiratory tract. Preferably, the present invention relates to the abovementioned use for preparing a pharmaceutical composition for treating asthma or COPD,

Within the scope of the present invention the compounds **1** and **2** may be administered simultaneously or successively, while it is preferable according to the invention to administer compounds **1** und **2** simultaneously.

The present invention further relates to the use of therapeutically effect amounts of salts **1** and betamimetics **2** for treating inflammatory or obstructive respiratory complaints, particularly asthma or COPD.

The proportions in which the active substances **1** and **2** may be used in the active substance combinations according to the invention are variable. Active substances **1** and **2** may possibly be present in the form of their solvates or hydrates. Depending on the choice of the compounds **1** and **2**, the weight ratios which may be used within the scope of the present invention vary on the basis of the different molecular weights of the various salt forms. The pharmaceutical combinations according to the invention may contain **1'** and **2'** generally in ratios by weight ranging from 1:5 to 500:1, preferably from 1:10 to 400:1,

The weight ratios specified below were based on the cation **1'** and the free bases **2'** of the betamimetics salmeterol and formoterol which are particularly preferred according to the invention.

The pharmaceutical combinations according to the invention may contain **1'** and **2'** in the case of formoterol, for example, in ratios by weight ranging from 1:10 to 300:1, preferably from 1:5 to 200:1, preferably 1:3 to 150:1, more preferably from 1:2 to 100:1. For example, without restricting the scope of the invention thereto, preferred combinations of **1** and **2** according to the invention may contain the cation **1'** and formoterol **2'** in the following weight ratios: 1:20,1:19,1:18,1:17,1:16,1:15,1:14,1:13,1:12,1:11,1:10,1:9, 1:8, 1:7,1:6,1:5,1:4,1:3,1:2,1:1,2:1, 3:1, 4:1,5:1, 6:1, 7:1, 8:1, 9:1, 10:1, 11:1, 12:1, 13:1,14:1, 15:1, 16:1, 17:1, 18:1, 19:1, 20:1, 21:1, 22:1, 23:1, 24:1, 25:1, 26;1, 27:1, 28:1, 29:1, 30:1, 31:1, 32:1, 33:1, 34:1, 35:1, 36:1, 37:1, 38:1, 39:1, 40:1, 41:1, 42:1, 43:1, 44:1, 45:1, 46:1, 47:1, 48:1, 49:1, 50:1, 51:1, 52:1, 53:1, 54:1, 55:1, 56:1, 57:1, 58:1, 59:1, 60:1, 61:1, 62:1, 63:1, 64:1, 65:1, 66:1, 67:1, 68:1, 69:1, 70:1, 71:1, 72:1, 73:1, 74:I, 75:1, 76:1, 77-1, 78:1, 79:1, 80:1, 81:1, 82:1, 83:1, 84:1, 85:1, 86:1, 87:1, 88:1, 89:1, 90:1, 91:1, 92:1, 93:1, 94:1, 95:1, 96;1, 97:1, 98:1, 99:1, 100:1
with the proviso that a solution (in mg/100 ml) consisting of :

| | |
|---|---|
| a compound of formula **1** | 333,3 mg /100 ml, |
| formoterol fumarate | 333,3 mg/100 ml , |
| benzalkoniumchloride 1 | 0,0 mg/100 ml , |
| EDTA, | 50,0 mg/100 ml and |
| HCl (1n) | ad pH 3.4. |

is excluded.

The pharmaceutical compositions according to the invention containing the combinations of **1** and **2** are normally administered so that the pharmacologically active cation **1'** and formoterol **2**' are present together in doses of 5 to 5000µg, preferably from 10 to 2000µg, more preferably from 15 to 1000µg, better still from 20 to 800µg, preferably, according to the invention, from 30 to 600µg, preferably from 40 to 500µg.

For example, combinations of **1** and **2** according to the invention contain a quantity of cation **1'** and formoterol **2**' such that the total dosage per single dose is about 10µg, 15µg, 20µg,25µg,30µg,35µg,45µg,50µg, 55µg,60µg,65µg,70µg,75µg,80µg,85µg,90µg, 95µg, 100µg, 105µg**,** 110µg, 115µg, 120µg, 125µg, 130µg, 135µg, 140µg, 145µg, 150µg, 155µg, 160µg, 165µg, 170µg, 175µg, 180µg, 185µg, 190µg, 195µg, 200µg, 205µg, 210µg, 215µg, 220µg, 225µg, 230µg, 235µg, 240µg, 245µg, 250µg, 255µg, 260µg, 265µg, 270µg, 275µg, 280µg, 285µg, 290µg, 295µg, 300µg, 305µg, 310µg, 315µg, 320µg, 325µg, 330µg, 335µg, 340µg, 345µg, 350µg, 355µg, 360µg, 365µg, 370µg, 375µg, 380µg, 385µg, 39µg, 395µg, 400µg, 405µg, 410µg, 415µg, 420µg, 425µg, 430µg, 435µg, 440µg, 445µg, 450µg, 455µg, 460µg, 465µg, 470µg, 475µg, 480µg, 485µg, 490µg, 495µg, 500µg, 505 µg, 510µg, 515µg, 520µg, 525µg, 530µg, 535µg, 540µg, 545µg, 495µg, 555µg, 560µg, 565µg, 570µg, 575µg, 580µg, 585µg, 590µg, 595µg, 600µg or similar. It is clear to anyone skilled in the art that the suggested dosages per single dose specified above are not to be regarded as being limited to the numerical values actually stated. Fluctuations of about ± 2.5 µg, particularly in the decimal range, are also included, as will be apparent to the skilled man. In these dosage ranges, the active substances **1'** and **2'** may be present in the weight ratios given above.

For example, without restricting the scope of the invention thereto, the combinations of **1** and **2** according to the invention may contain a quantity of cation **1'** and formoterol, **2'** such that, for each single dose, 8.2µg of **1'** and 2.5µg of **2'**, 8.2µg of **1'** and 4.9µg of **2',** 8.2µg of **1'** and 9.8µg of **2',** 8.2µg of **1'** and 14,7µg of **2',** 8.2µg of **1'** and 19.6µg of **2',** 8.2µg of **1'** and 24.4µg of **2',** 16.5µg of **1'** and 2.5µg of **2'**, 16.5µg of **1'** and 4.9µg of **2',** 16.5µg of **1'** and 9.8µg of **2',** 16.5µg of **1'** and 14.7µg of **2'** 16.5µg of **1'** and 19.6µg of **2',** 16.5µg of **1'** and 24.4µg of **2'**, 33.0µg of **1'** and 2.5µg of **2'** 33.0µg of **1'** and 4.9µg of **2',** 33.0µg of **1'** and 9.8µg of **2',** 33.0µg of **1'** and 14.7µg of **2'**, 33.0µg of **1'** and 19.6µg of **2',** 33.0µg of **1'** and 24.4µg of **2'**, 49.5µg of **1'** and 2.5µg of **2',** 49.5µg of **1'** and 4.9µg of **2'**, 49.5µg of **1'** and 9.8µg of **2',** 49.5µg of **1'** and 14.7µg of **2',** 49.5µg of **1'** and 19.6µg of **2',** 49.5µg of **1'** and 24.4µg of **2',** 82.5µg of **1'** and 2.5µg of **2'** 82.5µg of **1'** and 4.9µg of **2',** 82.5µg of **1'** and 9.8µg of **2',** 82.5µg of **1'** and 14.7µg of **2'**, 82.5µg of **1'** and 19.6µg of **2'**, 82.5µg of **1'** and 24.4µg of **2',** 165.0µg of **1'** and 2.5µg of **2',** 165.0µg of **1'** and 4.9µg of **2',** 165.0µg of **1'** and 9.8µg of **2',** 165.0µg of **1'** and 14.7µg of **2',** 165,0µg of **1'** and 19.6µg of **2',** 165.0µg of **1'** and 24.4µg of **2',** 206.2µg of **1'** and 2.5µg of **2'**, 206.2µg of **1'** and 4.9µg of **2',** 206.2µg of **1'** and 9.8µg of **2',** 206.2µg of **1'** and 14.7µg of **2',** 206.2µg of **1'** and 19.6µg of **2',** 206.2µg of **1'** and 24.4µg of **2',** 412.5µg of **1'** and 2.5µg of **2',** 412.5µg of **1'** and 4.9µg of **2'**, 412.5µg of **1'** and 9.8µg of **2'** 412.5µg of **1'** and 14.7µg of **2'**, 412.5µg of **1'** and 19.6µg of **2'**, 412.5µg of **1'** and 24.4µg of **2'** are present.

If the active substance combination in which the bromide is used as the salt **1** and in which **2** denotes formoterol fumarate is used as the preferred combination of **1'** and **2** according to the invention, the quantities of active substance **1**' and **2**' administered per single dose mentioned by way of example correspond to the following quantities of **1** and **2** administered per single dose: 10µg of **1** and 2.9µg of **2**, 10µg of **1** and 5.7µg of **2,** 10µg of **1** and 11.5µg of **2**, 10µg of **1** and 17.2µg of **2,** 10µg of **1** and 22.9µg of **2**, 10µg of **1** and 28.5µg of **2**, 20µg of **1** and 2.9µg of **2,** 20µg of **1** and 5.7µg of **2,** 20µg of **1** and 11.5µg of **2**, 20µg of **1** and 17.2µg of **2,** 20µg of **1** and 22.9µg of **2**, 20µg of **1** and 28:5µg of **2,** 40µg of **1** and 2.9µg of **2**, 40µg of **1** and 5.7µg of **2,** 40µg of **1** and 11,5µg of **2**, 40µg of **1** and 17.2µg of **2,** 40µg of **1** and 22.9µg of **2,** 40µg of **1** and 28.5µg of **2,** 60µg of **1** and 2.9µg of **2,** 60µg of **1** and 5.7µg of **2**, 60µg of **1** and 11.5µg of **2**, 60µg of **1** and 17.2µg of **2**, 60µg of **1** and 22.9µg of **2**, 60µg of **1** and 28.5µg of **2,** 100µg of **1** and 2.9µg of **2**, 100µg of **1** and 5.7µg of **2,** 100µg of **1** and 11.5µg of **2,** 10µg of **1** and 17.2µg of **2**, 100µg of **1** and 22.9µg of **2,** 100µg of **1** and 28.5µg of 200µg of **1** and 2.9µg of **2,** 200µg of **1** and 5.7µg of **2**, 200µg of **1** and 11.5µg of **2**, 200µg of **1** and 17.2µg of **2,** 200µg of **1** and 22.9µg of **2,** 200µg of **1** and 28.5µg of **2,** 250µg of **1** and 2.9µg of **2,** 250µg of **1** and 5.7µg of **2,** 250µg of **1** and 11.5µg of **2,** 250µg of **1** and 17.2µg of **2,** 250µg of **1** and 22.9µg of **2,** 250µg of **1** and 28.5µg of **2**, 500µg of **1** and 2.9µg of **2,** 500µg of **1** and 5.7µg of **2,** 500µg of **1** and 11.5µg of **2**, 500µg of **1** and 17.2µg of **2**, 500µg of **1** and 22.9µg of **2**, 500µg of **1** and 28.5µg of **2**.

If the active substance combination in which **2** denotes formoterol fumarate dihydrate and the salt **1** is bromide is used as a preferred combination of **1** and **2** according to the invention, the quantities of active substance **1**' and **2'** administered per single dose mentioned by way of example correspond to the following quantities of **1** and **2** administered per single dose: 10µg of **1** and 3µg of **2**, 10µg of **1** and 6µg of **2** 10µg of **1** and 12µg of **2**, 10µg of **1** and 18µg of **2**, 10µg of **1** and 24µg of **2**, 10µg of **1** and 30µg of **2,** 20µg of **1** and 3µg of **2,** 20µg of **1** and 6µg of **2**, 20µg of **1** and 12µg of **2,** 20µg of **1** and 18µg of **2,** 20µg of **1** and 24µg of **2**, 20µg of **1** and 30µg of **2**, 40µg of **1** and 3µg of **2,** 40µg of **1** and 6µg of **2**, 40µg of **1** and 12µg of **2**, 40µg of **1** and 18µg of **2**, 40µg of **1** and 24µg of **2**, 40µg of **1** and 30µg of **2**, 60µg of **1** and 3µg of **2,** 60µg of **1** and 6µg of **2**, 60µg of **1** and 12µg of **2**, 60µg of **1** and 18µg of **2**, 60µg of **1** and 24µg of **2**, 60µg of **1** and 30µg of **2**, 100µg of **1** and 3µg of **2**, 100µg of **1** and 6µg of **2**, 100µg of **1** and 12 µg of **2**, 100µg of **1** and 18µg of **2**, 100µg of **1** and 24µg of **2,** 100µg of **1** and 30µg of **2**, 200µg of **1** and 3µg of **2**, 200µg of **1** and 6µg of **2**, 200µg of **1** and 12 µg of **2**, 200µg of **1** and 18 µg of **2**, 200µg of **1** and 24µg of **2**, 200µg of **1** and 30µg of **2**, 250µg of **1** and 3µg of **2**, 250µg of **1** and 6µg of **2**, 250µg of **1** and 12µg of **2**, 250µg of **1** and 18µg of **2,** 250µg of **1** and 24µg of **2**, 250µg of **1** and 30µg of **2**, 500µg of **1** and 3µg of **2**, 500µg of **1** and 6µg of **2**, 500µg of **1** and 12µg of **2**, 500µg of **1** and 18µg of **2**, 500µg of **1** and 24µg of **2**, 500µg of **1** and 30µg of **2**.

The active substance combinations according to the invention may contain **1**' and **2'**, in the case of salmeterol, for example, in ratios by weight in the range from about 1:30 to 400:1, preferably 1:25 to 200:1, preferably 1:20 to 100:1, more preferably from 1:15 to 50:1.

For example, without restricting the scope of the invention thereto, preferred combinations of **1** and **2** according to the invention may contain the cation **1'** and salmeterol **2'** in the following ratios by weight: 1:15, 1:14, 1:13, 1:12, 1:11, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, 25:1, 26:1, 27:1, 28:1, 29:1, 30:1, 31:1, 32:1, 33:1, 34:1, 35:1
with the proviso that a solution (in mg/100 ml) consisting of :

| | |
|---|---|
| a compound of formula **1** | 333,3 mg /100 ml, |
| formoterol fumarate | 333,3 mg/100 ml, |
| benzalkoniumchloride | 10,0 mg/100 ml, |
| EDTA | 50,0 mg/100 ml and |
| HCl (1n) | ad pH 3.4 |

is excluded.

The pharmaceutical compositions according to the invention containing the combinations of **1** and **2** are usually administered so that the cation **1'** and salmeterol **2'** are present together in dosages of 5 to 5000µg, preferably from 10 to 2000µg, more preferably from 15 to 1000µg, even more preferably from 20 to 800µg, and preferably according to the invention from 30 to 750µg, preferably from 40 to 700µg per single dose.

For example, combinations of **1** and **2** according to the invention contain an amount of **1'** and salmeterol **2'** such that the total dosage per single dose is about 15µg, 20µg, 25µg, 30µg, 35µg, 45µg, 50µg, 55µg, 60µg, 65µg, 70µg, 75µg, 80µg, 85µg, 90µg, 95µg, 100µg, 105µg, 110µg, 115µg, 120µg, 125µg, 130µg, 135µg, 140µg, 145µg, 150µg, 155µg, 160µg, 165µg, 170µg, 175µg, 180µg, 185µg, 190µg, 195µg, 200µg, 205µg, 210µg, 215µg, 220µg, 225µg, 230µg, 235µg, 240µg, 245µg, 250µg, 255µg, 260µg, 265µg, 270µg, 275µg, 280µg, 285µg, 290µg, 295µg, 300µg, 305µg, 310µg, 315µg, 320µg, 325µg, 330µg, 335µg, 340µg, 345µg, 350µg, 355µg, 360µg, 365µg, 370µg, 375µg, 380µg, 385µg, 390µg, 395µg, 400µg, 405µg, 410µg, 415µg, 420µg, 425µg, 430µg, 435µg, 440µg, 445µg, 450µg, 455µg, 460µg, 465µg, 470µg, 475µg, 480µg, 485µg, 490µg, 495µg, 500µg, 505µg, 510µg, 515µg, 520µg, 525µg, 530µg, 535µg, 540µg, 545µg, 550µg, 555µg, 560µg, 565µg, 570µg, 575µg, 580µg, 585µg, 590µg, 595µg, 600µg, 605µg, 610µg, 615µg, 620µg, 625µg, 630µg, 635µg, 640µg, 645µg, 650µg, 655µg, 660µg, 665µg, 670µg, 675µg, 680µg, 685µg, 690µg, 695µg, 700µg or similar. It is clear to anyone skilled in the art that the suggested dosages per single dose specified above are not to be regarded as being limited to the numerical values actually stated. Fluctuations of about ± 2.5 µg, particularly in the decimal range, are also included, as will be apparent to the skilled man. In these dosage ranges, the active substances **1'** and **2'** may be present in the weight ratios given above.

For example, without restricting the scope of the invention thereto, the combinations of **1** and 2 according to the invention may contain a quantity of cation **1'** and salmeterol **2'** such that, for each single dose, 8.2µg of **1'** and 12.5µg of **2'**, 8.2µg of **1'** and 25µg of **2'**, 8.2µg of **1'** and 50µg of **2'** 8.2µg of **1'** and 75µg of **2'**, 8.2µg of **1'** and 100µg of **2'**, 8.2µg of **1'** and 200µg of **2'**, 16.5µg of **1'** and 12.5µg of **2'**, 16.5µg of **1'** and 25µg of **2'**, 16.5µg of **1'** and 50µg of **2'**, 16.5µg of **1'** and 75µg of **2'**, 16.5µg of **1'** and 100µg of **2'**, 16.5µg of **1'** and 200µg of **2'**, 33.0µg of **1'** and 12.5µg of **2'**, 33.0µg of **1'** and 25µg of **2'**, 33.0µg of **1'** and 50µg of **2'**, 33.0µg of **1'** and 75µg of **2'**, 33.0µg of **1'** and 100µg of **2'** 33.0µg of **1'** and 200µg of **2'**, 49.5µg of **1'** and 12.5µg of **2'**, 49.5µg of **1'** and 25µg of **2'**, 49.5µg of **1'** and 50µg of **2'**, 49.5µg of **1'** and 75µg of **2'**, 49.5µg of **1'** and 100µg of **2'**, 49.5µg of **1'** and 200µg of **2'**, 82.5µg of **1'** and 12.5µg of **2'**, 82.5µg of **1'** and 25µg of **2'**, 82.5µg of **1'** and 50µg of **2'**, 82.5µg of **1'** and 75µg of **2'**, 82.5µg of **1'** and 100µg of **2'**, 82.5µg of **1'** and 200 µg of **2'**, 165.0µg of **1'** and 12.5µg of **2'**, 165.0µg of **1'** and 25µg of **2'**, 165.0µg of **1'** and 50µg of **2'**, 165.0µg of **1'** and 75µg of **2'**, 165.0µg of **1'** and 100µg of **2'**, 165.0µg of **1'** and 200µg of **2'**, 206.2µg of **1'** and 12.5µg of **2'**, 206.2µg of **1'** and 25µg of **2'**, 206.2µg of **1'** and 50µg of **2'**, 206.2µg of **1'** and 75µg of **2'**, 206.2µg of **1'** and 100µg of **2'**, 206.2µg of **1'** and 200µg of **2'**, 412.5µg of **1'** and 12.5µg of **2'**, 412.5µg of **1'** and 25µg of **2'**, 412.5µg of **1'** and 50µg of **2'**, 412.5µg of **1'** and 75µg of **2'**, 412,5µg of **1'** and 100µg of **2'**, 412.5µg of **1'** and 200µg of **2'** are present, for example.

If a combination of active substances wherein the bromide is used as the salt **1** and **2** denotes salmeterol xinafoate is used as the preferred combination of **1** and **2** according to the invention, the amounts of active substances **1'** and **2'** administered per single dose as specified hereinbefore correspond to the following amounts of **1** and **2** administered per single dose: 10µg of **1** and 18.2µg of **2,** 10µg of **1** and 36.3µg of **2,** 10µg of **1** and 72.6µg of **2,** 10µg of **1** and 108.9µg of **2,** 10µg of **1** and 145.2µg of **2,** 10µg of **1** and 290.4µg of **2,** 20µg of **1** and 18.2µg of **2,** 20µg of **1** and 36.3µg of **2,** 20µg of **1** and 72.6µg of **2,** 20µg of **1** and 108.9µg of **2,** 20µg of **1** and 145.2µg of **2,** 20µg of **1** and 290.4µg of **2,** 40µg of **1** and 18.2µg of **2,** 40µg of **1** and 36.3µg of **2,** 40µg of **1** and 72.6µg of **2,** 40µg of **1** and 108.9µg of **2,** 40µg of **1** and 145.2µg of **2,** 40µg of **1** and 290.4µg of **2,** 60µg of **1** and 18.2µg of **2,** 60µg of **1** and 36.3µg of **2,** 60µg of 1 and 72.6µg of **2,** 60µg of **1** and 108.9µg of **2,** 60µg of **1** and 145.2µg of **2,** 60µg of **1** and 290.4µg of **2,** 100µg of **1** and 18.2µg of **2,** 100µg of **1** and 36.3µg of **2,** 100µg of **1** and 72.6µg of **2,** 100µg of **1** and 108.9µg of **2,** 100µg of **1** and 145.2µg of **2,** 100µg of **1** and 290.4µg of **2,** 200µg of **1** and 18.2µg of **2,** 200µg of **1** and 36.3µg of **2,** 200µg of **1** and 72.6µg of **2,** 200µg of **1** and 108.9µg of **2,** 200µg of **1** and 145.2µg of **2,** 200µg of **1** and 290.4µg of **2,** 250µg of **1** and 18.2µg of **2,** 250µg of **1** and 36.3µg of **2,** 250µg of **1** and 72.6µg of **2,** 250µg of **1** and 108.9µg of **2,** 250µg of **1** and 145.2µg of **2,** 250µg of **1** and 290.4µg of **2,** 500µg of **1** and 18.2µg of **2,** 500µg of **1** and 36.3µg of **2,** 500µg of **1** and 72.6µg of **2,** 500µg of **1** and 108.9µg of **2,** 500µg of **1** and 145.2µg of **2,** 500µg of **1** and 290.4µg of **2.**

The quantities of active substance in the pharmaceutical combinations according to the invention which are administered per single dose can be calculated analogously if instead of the salmeterol xinafoate the compounds **2** salmeterol-4-phenylcinnamic acid salt (4-phenylcinnamate) and salmeterol-5-(2,4-difluorophenyl)salicylic acid salt (5-(2,4-difluorophenyl)salicylate) which are also preferably used according to the invention are used.

The aforementioned examples of possible doses applicable for the combinations according to the invention are to be understood as referring to doses per single application. However, these examples are not be understood as excluding the possibility of administering the combinations according to the invention multiple times. Depending on the medical need patients may receive also multiple inhalative applications. As an example patients may receive the combinations according to the invention for instance two or three times (e.g. two or three puffs) in the morning of each treatment day. As the aforementioned dose examples are only to be understood as dose examples per single application (i.e. per puff) multiple application of the combinations according to the invention leads to multiple doses of the aforementioned examples. The application of the compositions according to the invention can be for instance once a day, or depending on the duration of action of the anticholinergic agent twice a day, or once every 2 or 3 days.

Moreover it is emphazised that the aforementioned dose examples are to be understood as examples of metered doses only. In other terms, the aforementioned dose examples are not to be understood as the effective doses of the combinations according to the invention that do in fact reach the lung. It is clear for the person of ordinary skill in the art that the delivered dose to the lung is generally lower than the metered dose of the administered active ingredients.

The active substance combinations of **1** and **2** according to the invention are administered by inhalation, as propellant-free inhalable solutions. Within the scope of the present invention, the term propellant-free inhalable solutions also includes concentrates or sterile inhalable solutions ready for use. The preparations according to the invention may contain the combination of active substances **1** and **2** either together in one formulation or in two separate formulations. These formulations which may be used within the scope of the present invention are described in more detail in the next part of the specification.

**Propellant-free inhalable solutions or suspensions containing the combinations of active substances 1 and 2 according to the invention:**
Propellant-free inhalable solutions and suspensions according to the invention contain, for example, aqueous or alcoholic, preferably ethanolic solvents, optionally ethanolic solvents mixed with aqueous solvents. If aqueous/ethanolic solvent mixtures are used the relative proportion of ethanol compared with water is not limited but preferably the maximum is up to 70 percent by volume, more particularly up to 60 percent by volume of ethanol. The remainder of the volume is made up of water. The solutions or suspensions containing **1** and **2,** separately or together, are adjusted to a pH of 2 to 7, preferably 2 to 5, using suitable acids. The pH may be adjusted using acids selected from inorganic or organic acids. Examples of particularly suitable inorganic acids include hydrochloric acid, hydrobromic acid, nitric acid, sulphuric acid and/or phosphoric acid. Examples of particularly suitable organic acids include ascorbic acid, citric acid, malic acid, tartaric acid, maleic acid, succinic acid, fumaric acid, acetic acid, formic acid and/or propionic acid etc. Preferred inorganic acids are hydrochloric and sulphuric acids. It is also possible to use the acids which have already formed an acid addition salt with one of the active substances. Of the organic acids, ascorbic acid, fumaric acid and citric acid are preferred. If desired, mixtures of the above acids may be used, particularly in the case of acids which have other properties in addition to their acidifying qualities, e.g. as flavourings, antioxidants or complexing agents, such as citric acid or ascorbic acid, for example. According to the invention, it is particularly preferred to use hydrochloric acid to adjust the pH.

According to the invention, the addition of editic acid (EDTA) or one of the known salts thereof, sodium editate, as stabiliser or complexing agent is unnecessary in the present formulation. Other embodiments may contain this compound or these compounds. In a preferred embodiment the content based on sodium editate is less than 100mg/100ml, preferably less than 50mg/100 ml, more preferably less than 20mg/100 ml. Generally, inhalable solutions in which the content of sodium editate is from 0 to 10mg/100ml are preferred.

Co-solvents and/or other excipients may be added to the propellant-free inhalable solutions according to the invention. Preferred co-solvents are those which contain hydroxyl groups or other polar groups, e.g. alcohols - particularly isopropyl alcohol, glycols - particularly propyleneglycol, polyethyleneglycol, polypropyleneglycol, glycolether, glycerol, polyoxyethylene alcohols and polyoxyethylene fatty acid esters. The terms excipients and additives in this context denote any pharmacologically acceptable substance which is not an active substance but which can be formulated with the active substance or substances in the pharmacologically suitable solvent in order to improve the qualitative properties of the active substance formulation. Preferably, these substances have no pharmacological effect or, in connection with the desired therapy, no appreciable or at least no undesirable pharmacological effect. The excipients and additives include, for example, surfactants such as soya lecithin, oleic acid, sorbitan esters, such as polysorbates, polyvinylpyrrolidone, other stabilisers, complexing agents, antioxidants and/or preservatives which guarantee or prolong the shelf life of the finished pharmaceutical formulation, flavourings, vitamins and/or other additives known in the art. The additives also include pharmacologically acceptable salts such as sodium chloride as isotonic agents. The preferred excipients include antioxidants such as ascorbic acid, for example, provided that it has not already been used to adjust the pH, vitamin A, vitamin E, tocopherols and similar vitamins and provitamins occurring in the human body.

Preservatives may be used to protect the formulation from contamination with pathogens. Suitable preservatives are those which are known in the art, particularly cetyl pyridinium chloride, benzalkonium chloride or benzoic acid or benzoates such as sodium benzoate in the concentration known from the prior art. The preservatives mentioned above are preferably present in concentrations of up to 50mg/100ml, more preferably between 5 and 20mg/100ml.

Preferred formulations contain, in addition to the solvent water and the combination of active substances **1** and **2,** only benzalkonium chloride and sodium editate. In another preferred embodiment, no sodium editate is present

The propellant-free inhalable solutions according to the invention are administered in particular using inhalers of the kind which are capable of nebulising a small amount of a liquid formulation in the therapeutic dose within a few seconds to produce an aerosol suitable for therapeutic inhalation. Within the scope of the present invention, preferred inhalers are those in which a quantity of less than 100µL, preferably less than 50µL, more preferably between 20 and 30µL of active substance solution can be nebulised in preferably one spray action to form an aerosol with an average particle size of less than 20µm, preferably less than 10µm, in such a way that the inhalable part of the aerosol corresponds to the therapeutically effective quantity.

An apparatus of this kind for propellant-free delivery of a metered quantity of a liquid pharmaceutical composition for inhalation is described for example in International Patent Application WO 91/14468 and also in WO 97/12687 (cf. in particular Figures 6a and 6b). The nebulisers (devices) described therein are known by the name Respimat®.
This nebuliser (Respimat®) can advantageously be used to produce the inhalable aerosols according to the invention containing the combination of active substances **1** and **2**. Because of its cylindrical shape and handy size of less than 9 to 15 cm long and 2 to 4 cm wide, this device can be carried at all times by the patient. The nebuliser sprays a defined volume of pharmaceutical formulation using high pressures through small nozzles so as to produce inhalable aerosols.

The preferred atomiser essentially consists of an upper housing part, a pump housing, a nozzle, a locking mechanism, a spring housing, a spring and a storage container, characterised by
- a pump housing which is secured in the upper housing part and which comprises at one end a nozzle body with the nozzle or nozzle arrangement,
- a hollow plunger with valve body,
- a power takeoff flange in which the hollow plunger is secured and which is located in the upper housing part,
- a locking mechanism situated in the upper housing part,
- a spring housing with the spring contained therein, which is rotatably mounted on the upper housing part by means of a rotary bearing,
- a lower housing part which is fitted onto the spring housing in the axial direction.

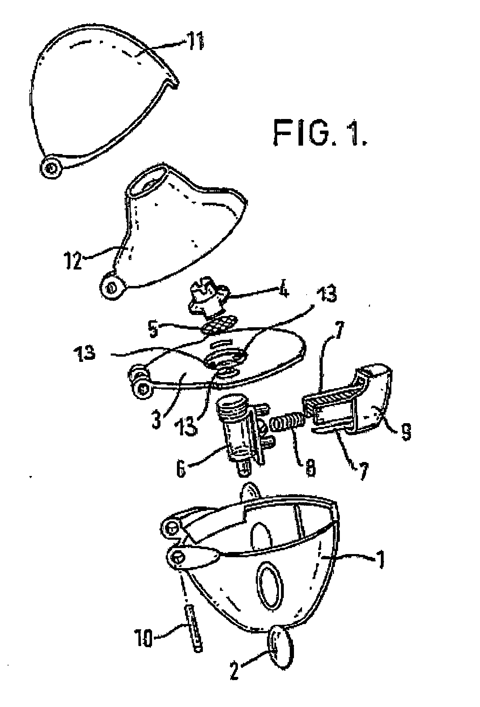

The hollow plunger with valve body corresponds to a device disclosed in WO 97/12687. It projects partially into the cylinder of the pump housing and is axially movable within the cylinder. Reference is made in particular to Figures 1 to 4, especially Figure 3, and the relevant parts of the description. The hollow plunger with valve body exerts a pressure of 5 to 60 Mpa (about 50 to 600 bar), preferably 10 to 60 Mpa (about 100 to 600 bar) on the fluid, the measured amount of active substance solution, at its high pressure end at the moment when the spring is actuated. Volumes of 10 to 50 microlitres are preferred, while volumes of 10 to 20 microlitres are particularly preferred and a volume of 15 microlitres per spray is most particularly preferred.

The valve body is preferably mounted at the end of the hollow plunger facing the valve body.

The nozzle in the nozzle body is preferably microstructured, i.e, produced by microtechnology. Microstructured nozzle bodies are disclosed for example in WO 94/07607; reference is hereby made to the contents of this specification, particularly Figure 1 therein and the associated description.

The nozzle body consists for example of two sheets of glass and/or silicon firmly joined together, at least one of which has one or more microstructured channels which connect the nozzle inlet end to the nozzle outlet end. At the nozzle outlet end there is at least one round or non-round opening 2 to 10 microns deep and 5 to 15 microns wide, the depth preferably being 4.5 to 6.5 microns while the length is preferably 7 to 9 microns.
In the case of a plurality of nozzle openings, preferably two, the directions of spraying of the nozzles in the nozzle body may extend parallel to one another or may be inclined relative to one another in the direction of the nozzle opening. In a nozzle body with at least two nozzle openings at the outlet end the directions of spraying may be at an angle of 20 to 160° to one another, preferably 60 to 150°, most preferably 80 to 100°. The nozzle openings are preferably arranged at a spacing of 10 to 200 microns, more preferably at a spacing of 10 to 100 microns, most preferably 30 to 70 microns. Spacings of 50 microns are most preferred. The directions of spraying will therefore meet in the vicinity of the nozzle openings.

The liquid pharmaceutical preparation strikes the nozzle body with an entry pressure of up to 600 bar, preferably 200 to 300 bar, and is atomised into an inhalable aerosol through the nozzle openings. The preferred particle or droplet sizes of the aerosol are up to 20 microns, preferably 3 to 10 microns.

The locking mechanism contains a spring, preferably a cylindrical helical compression spring, as a store for the mechanical energy. The spring acts on the power takeoff flange as an actuating member the movement of which is determined by the position of a locking member. The travel of the power takeoff flange is precisely limited by an upper and lower stop. The spring is preferably biased, via a power step-up gear, e.g. a helical thrust gear, by an external torque which is produced when the upper housing part is rotated counter to the spring housing in the lower housing part. In this case, the upper housing part and the power takeoff flange have a single or multiple V-shaped gear.

The locking member with engaging locking surfaces is arranged in a ring around the power takeoff flange. It consists, for example, of a ring of plastic or metal which is inherently radially elastically deformable. The ring is arranged in a plane at right angles to the atomiser axis. After the biasing of the spring, the locking surfaces of the locking member move into the path of the power takeoff flange and prevent the spring from relaxing. The locking member is actuated by means of a button. The actuating button is connected or coupled to the locking member. In order to actuate the locking mechanism, the actuating button is moved parallel to the annular plane, preferably into the atomiser; this causes the deformable ring to deform in the annular plane. Details of the construction of the locking mechanism are given in WO 97/20590.

The lower housing part is pushed axially over the spring housing and covers the mounting, the drive of the spindle and the storage container for the fluid.
When the atomiser is actuated the upper housing part is rotated relative to the lower housing part, the lower housing part taking the spring housing with it. The spring is thereby compressed and biased by means of the helical thrust gear and the locking mechanism engages automatically. The angle of rotation is preferably a whole-number fraction of 360 degrees, e.g. 180 degrees. At the same time as the spring is biased, the power takeoff part in the upper housing part is moved along by a given distance, the hollow plunger is withdrawn inside the cylinder in the pump housing, as a result of which some of the fluid is sucked out of the storage container and into the high pressure chamber in front of the nozzle.

If desired, a number of exchangeable storage containers which contain the fluid to be atomised may be pushed into the atomiser one after another and used in succession. The storage container contains the aqueous aerosol preparation according to the invention.
The atomising process is initiated by pressing gently on the actuating button. As a result, the locking mechanism opens up the path for the power takeoff member. The biased spring pushes the plunger into the cylinder of the pump housing. The fluid leaves the nozzle of the atomiser in atomised form.

Further details of construction are disclosed in PCT Applications WO 97/12683 and WO 97/20590, to which reference is hereby made.
The components of the atomiser (nebuliser) are made of a material which is suitable for its putpose. The housing of the atomiser and, if its operation permits, other parts as well, are preferably made of plastics, e.g. by injection moulding. For medicinal purposes, physiologically safe materials are used.

Figures 6a/b of WO 97/12687, show the nebuliser (Respimat®) which can advantageously be used for inhaling the aqueous aerosol preparations according to the invention.
Figure 6a of WO 97/12687 shows a longitudinal section through the atomiser with the spring biased while Figure 6b of WO 97/12687 shows a longitudinal section through the atomiser with the spring relaxed.
The upper housing part (51) contains the pump housing (52) on the end of which is mounted the holder (53) for the atomiser nozzle. In the holder is the nozzle body (54) and a filter (55). The hollow plunger (57) fixed in the power takeoff flange (56) of the locking mechanism projects partially into the cylinder of the pump housing. At its end the hollow plunger carries the valve body (58). The hollow plunger is sealed off by means of the seal (59). Inside the upper housing part is the stop (60) on which the power takeoff flange abuts when the spring is relaxed. On the power takeoff flange is the stop (61) on which the power takeoff flange abuts when the spring is biased. After the biasing of the spring the locking member (62) moves between the stop (61) and a support (63) in the upper housing part. The actuating button (64) is connected to the locking member. The upper housing part ends in the mouthpiece (65) and is sealed off by means of the protective cover (66) which can be placed thereon.
The spring housing (67) with compression spring (68) is rotatably mounted on the upper housing part by means of the snap-in lugs (69) and rotary bearing. The lower housing part (70) is pushed over the spring housing. Inside the spring housing is the exchangeable storage container (71) for the fluid (72) which is to be atomised. The storage container is sealed off by the stopper (73) through which the hollow plunger projects into the storage container and is immersed at its end in the fluid (supply of active substance solution).
The spindle (74) for the mechanical counter is mounted in the covering of the spring housing. At the end of the spindle facing the upper housing part is the drive pinion (75). The slider (76) sits on the spindle.

The nebuliser described above is suitable for nebulising the aerosol preparations according to the invention to produce an aerosol suitable for inhalation.
If the formulation according to the invention is nebulised using the method described above (Respimat®) the quantity delivered should correspond to a defined quantity with a tolerance of not more than 25%, preferably 20% of this amount in at least 97%, preferably at least 98% of all operations of the inhaler (spray actuations). Preferably, between 5 and 30 mg of formulation, most preferably between 5 and 20 mg of formulation are delivered as a defined mass on each actuation.

However, the formulation according to the invention may also be nebulised by means of inhalers other than those described above, e.g. jet stream inhalers or other stationary nebulisers.

Accordingly, in a further aspect, the invention relates to pharmaceutical formulations in the form of propellant-free inhalable solutions or suspensions as described above combined with a device suitable for administering these formulations, preferably in conjunction with the Respimat®. Preferably, the invention relates to propellant-free inhalable solutions or suspensions characterised by the combination of active substances **1** and **2** according to the invention in conjunction with the device known by the name Respimat®. In addition, the present invention relates to the above-mentioned devices for inhalation, preferably the Respimat®, characterised in that they contain the propellant-free inhalable solutions or suspensions according to the invention as described hereinbefore.

According to the invention, inhalable solutions which contain the active substances **1** and **2** in a single preparation are preferred. The term "single preparation" also includes preparations which contain the two ingredients **1** and **2** in two-chamber cartridges, as disclosed for example in WO 00/23037. Reference is hereby made to this publication in its entirety.

The propellant-free inhalable solutions or suspensions according to the invention may take the form of concentrates or sterile inhalable solutions or suspensions ready for use, as well as the above-mentioned solutions and suspensions designed for use in a Respimat®. Formulations ready for use may be produced from the concentrates, for example, by the addition of isotonic saline solutions. Sterile formulations ready for use may be administered using energy-operated free-standing or portable nebulisers which produce inhalable aerosols by means of ultrasound or compressed air by the Venturi principle or other principles.

Accordingly, in another aspect, the present invention relates to pharmaceutical compositions in the form of propellant-free inhalable solutions or suspensions as described hereinbefore which take the form of concentrates or sterile formulations ready for use, combined with a device suitable for administering these solutions, characterised in that the device is an energy-operated free-standing or portable nebuliser which produces inhalable aerosols by means of ultrasound or compressed air by the Venturi principle or other methods.

The Examples which follow serve to illustrate the present invention in more detail without restricting the scope of the invention to the following embodiments by way of example. First, the preparation of compounds 1 which are not known in the art will be described.

### 1) Preparation of the compounds of formula 1 (in form of the bromide salt):

### 1.1.: 9-methyl-fluorene-9-carboxylic acid:

### a) methyl 9-methyl-fluorene-9-carboxylate:

From 7.6 g (0.33 mol) of sodium and 300 ml of ethanol a sodium ethoxide solution is prepared, to which 69.6 g (0.33 ml) of 9-fluorenecarboxylic acid are added batchwise. After the addition has ended the mixture is stirred for 2,5 hours at ambient temperature. Then it is evaporated to dryness, the residue is suspended in 600 ml of dimethylformamide and 93.96 g (0.662 mol) of methyl iodide are added dropwise. The mixture is stirred for 3 hours at constant temperature. The cloudy solution is stirred into 500 ml of water and 300 ml of diethyl ether with cooling and extracted, the organic phase is washed with water and 10% sodium carbonate solution, dried and evaporated to dryness. The residue is purified by column chromatography, eluant: cyclohexane / ethyl acetate 96:4.
Yield: 12.61 g of white crystals (= 16% of theoretical); melting point: 108°-109°C.

### b) 9-methyl-fluorene-9-carboxylic acid:

12.6 g (0.053 mol) of methyl 9-methyl-fluorene-9-carboxylate and 53 ml of 2 molar, aqueous sodium hydroxide solution are stirred in 120 ml of 1,4-dioxane for 24 hours at ambient temperature. The dioxane is distilled off, made up to a total volume of 300 ml with water and extracted with diethyl ether. The aqueous phase is acidified with 3 molar, aqueous HCl, crystallised and filtered.
Yield: 11.25 g of white crystals (= 95% of theoretical); melting point: 168°-169°C.

### 1.2: tropenol 9-methyl-fluorene-9-carboxylate:

6.73 g (0.03 mol) of 9-methyl-fluorene-9-carboxylic acid are suspended in 60 ml dichloromethane, combined with 5.0 g of oxalyl chloride and 1 drop of dimethylformamide, then stirred for one hour at ambient temperature and finally the solvent is distilled off. The acid chloride remaining is used in the next step without any further purification. 4.18 g (0.03 mol) of tropenol and 4.27 g (0.033 mol) of diisopropylethylamine are suspended in 100 ml of dichloroethane, the acid chloride is added dropwise to 30 ml of dichloroethane at 35-40°C and then stirred for 24 hours at 40° C. The suspension is diluted with dichloromethane and extracted with dilute hydrochloric acid. The organic phase is then washed with water, dried over MgSO₄ and the product is converted into its hydrochloride with a solution of HCl in diethyl ether. The solvent is then removed. To purify it the precipitated hydrochloride is taken up in water and extracted with diethyl ether. The aqueous phase is made basic with 10% aq. sodium carbonate solution and extracted with dichloromethane. The organic phase is dried over MgSO₄ and the solvent is distilled off.
Yield: 4.40 g of yellow oil (= 42% of theoretical);

### 1.3: scopine 9-methyl-fluorene-9-carboxylate:

2.5 g (0.007 mol) of tropenol 9-methyl-fluorene-9-carboxylate are suspended in about 25 ml of dimethylformamide and combined with 0.13 g (0.001 mol) of vanadium-(V)-oxide. At 60°C a solution of 1.43 g (0.015 mol) of H₂O₂-urea in about 5.5 ml of water is added dropwise and stirred for 6 hours at 60°C. After cooling to 20°C the precipitate formed is suction filtered, the filtrate is adjusted to pH 2 with 4 N hydrochloric acid and combined with Na₂S₂O₅ dissolved in water- The resulting solution is evaporated to dryness, the residue is extracted with dichloromethane/water. The acidic aqueous phase is made basic with Na₂CO₃, extracted with dichloromethane and the organic phase is dried over Na₂SO₄ and concentrated. Then about 0.4 ml of acetylchloride is added at ambient temperature and the mixture is stirred for 1 hour. After extraction with 1 N hydrochloric acid the aqueous phase is made basic, extracted with dichloromethane, the organic phase is washed with water and dried over Na₂SO₄. Then the solvent is removed by distillation. The crude product is purified by recrystallisation from diethyl ether.
Yield: 1.8 g of white crystals (= 71 % of theoretical).

### 1.4. scopine 9-methyl-fluorene-9-carboxylate methobromide :

1.8 g (0.005 mol) of scopine 9-methyl-fluorene-9-carboxylate are taken up in 30 ml acetonitrile and reacted with 2.848 g (0.015 mol) of 50% methyl bromide solution in acetonitrile. The reaction mixture is left to stand for 3 days at ambient temperature, during which time the product crystallises. The crystals precipitated are separated off and recrystallised from diethyl ether to purify them.
Yield: 1.6 g of white crystals (= 70 % of theoretical); melting point: 214°C.

| Elemental analysis: | | | |
|---|---|---|---|
| calculated: | C (62.13) | H (5.93) | N (4.26) |
| found: | C (62.23) | H (6.05) | N (4.32). |

## Claims

1. Propellant-free inhalable solutions or suspensions containing one or more salts of formula **1** 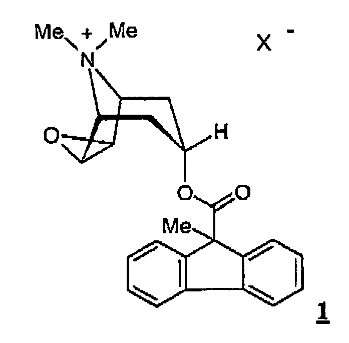 wherein
X⁻ denotes an anion with a single negative charge, preferably an anion selected from the group consisting of fluoride, chloride, bromide, iodide, sulphate, phosphate, methanesulphonate, nitrate, maleate, acetate, citrate, fumarate, tartrate, oxalate, succinate, benzoate and p-toluenesulphonate,
combined with one or more betamimetics (**2**), optionally in the form of the enantiomers, mixtures of the enantiomers or in the form of the racemates thereof, optionally in the form of the solvates or hydrates, further containing a solvent selected from water, ethanol and a mixture of water and ethanol and optionally further containing a pharmaceutically acceptable excipient,
with the proviso that a solution (in mg/100 ml) consisting of:
| | |
|---|---|
| a compound of formula **1** | 333,3 mg/100ml, |
| formoterol fumarate | 333,3 mg/100 ml, |
| benzalkoniumchloride | 10,0 mg/100 ml, |
| EDTA | 50,0 mg/100 ml and |
| HCl (In) | ad pH 3.4 |
is excluded.

2. Propellant-free inhalable solutions or suspensions according to claim 1, **characterised in that** the betamimetics **2** are selected from the group consisting of albuterol, bambuterol, bitolterol, broxaterol, carbuterol, clenbuterol, fenoterol, formoterol, hexoprenaline, ibuterol, isoetharine, isoprenaline, levosalbutamol, mabuterol, meluadrine, metaproterenol, orciprenaline, pirbuterol, procaterol, reproterol, rimiterol, ritodrine, salmeterol, salmefamol, soterenot, sulphonterol, tiaramide, terbutaline, tolubuterol, CHF-1035, HOKU-81, KUL-1248, 3-(4-{6-[2-Hydroxy-2-(4-hydroxy-3-hydroxymethylphenyl)-ethylamino]-hexyloxy}-butyl)-benzenesulfoneamide, 5-[2-(5,6-Diethyl-indan-2-ylamino)-1-hydroxy-ethyl]-8-hydroxy-1*H*-quinolin-2-one, 4-hydroxy-7-[2-{[2-{[3-(2-phenylethoxy)propyl]sulphonyl}ethyl]-amino}ethyl]-2(3H)-benzothiazolone, 1-(2-fluoro-4-hydroxyphenyl)-2-[4-(1-benzimidazolyl)-2-methyl-2-butylamino]ethanol, 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-{4-methoxyphenyl)-1,2,4-triazol-3-yl]-2-methyl-2-butylaminol}ethanol, 5-hydroxy-8-(1-hydroxy-2-isopropylaminobutyl)-2H-1,4-benzoxazin-3-(4H)-one, 1-(4-amino-3-chloro-5-trifluormethylphenyl)-2-tert-butylamino)ethanol and 1-(4-ethoxycarbonylamino-3-cyano-5-fluorophenyl)-2-(tert.-butylamino)ethanol, optionally in the form of the racemates, the enantiomers, the diastereomers and optionally the pharmacologically acceptable acid addition salts and the hydrates thereof.

3. Propellant-free inhalable solution or suspensions according to one of claims 1 or 2, **characterised in that** the compounds **2** are present in the form of the salts of hydrochloric acid, hydrobromic acid, sulphuric acid, phosphoric acid, methanesulphonic acid, acetic acid, fumaric acid, succinic acid, lactic acid, citric acid, tartaric acid, 1-hydroxy-2-naphthalenecarboxylic acid, 4-phenylcinnamic acid, 5-(2.4-difluorophenyl)salicylic acid or maleic acid or mixtures thereof.

4. Propellant-free inhalable solutions or suspensions according to one of claims 1 to 3, **characterised in that** the solution is adjusted to a pH of 2 to 7 with an organic or inorganic acid.

5. Propellant-free inhalable solution s or suspensions according to claim 4, **characterised in that** the solution is adjusted to a pH of 2 to 5 with an organic or inorganic acid.

6. Propellant-free inhalable solutions or suspensions according to one of claims 1 to 3, **characterised in that** the weight ratios of **1'** 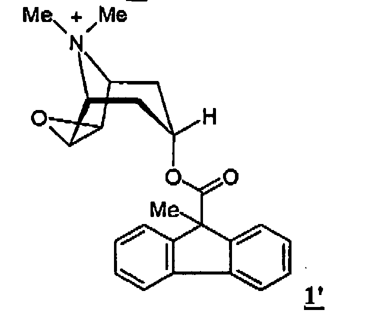 to **2**' are in a range from about 1:5 to 500:1, preferably 1:10 to 400:1.

7. Propellant-free inhalable solutions or suspensions according to one of claims 1 to 6, **characterised in that** the weight ratios of **1'** to salmeterol **2'** are in a range from about 1:30 to 400:1, preferably 1:25 to 200:1.

8. Propellant-free inhalable solutions or suspensions according to one of claims 1 to 7, **characterised in that** the weight ratios of **1'** 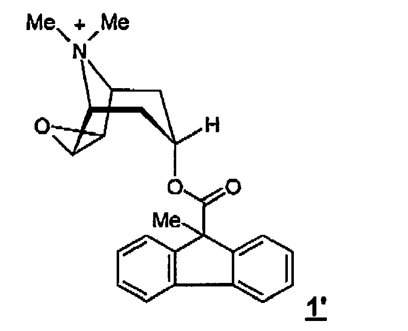 to formoterol **2'** are in a range from about 1:10 to 300:1, preferably 1:5 to 200:1

9. Propellant-free inhalable solutions or suspensions according to one of claims 1 to 8, **characterised in that** it contains other co-solvents and/or excipients.

10. Propellant-free inhalable solutions or suspensions according to claim 9, **characterised in that** it contains as co-solvents ingredients which contain hydroxyl groups or other polar groups, e.g. alcohols - particularly isopropyl alcohol, glycols - particularly propyleneglycol polyethyleneglycol, polypropyleneglycol, glycolether, glycerol, polyoxyethylene alcohols and polyoxyethylene fatty acid esters.

11. Propellant-free inhalable solutions or suspensions according to one of claims 9 to 10, **characterised in that** they contain as excipients surfactants, stabilisers, antioxidants and/or preservatives, flavourings, pharmacologically acceptable salts and/or vitamins.

12. Propellant-free inhalable solutions or suspensions according to claim 1, **characterised in that** they contain as complexing agent sodium edetate,

13. Use of a propellant-free inhalable solutions or suspensions according to one of claims 1 to 12 for preparing a medicament for the treatment of inflammatory or obstructive respiratory complaints, particularly asthma or COPD.

## Patentansprüche

1. Treibmittelfreie inhalierbare Lösungen oder Suspensionen, enthaltend ein oder mehr Salze der Formel **1** 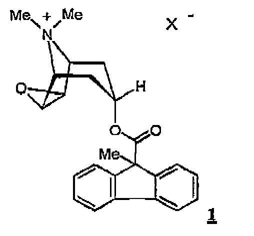 worin
X⁻ ein Anion mit einer einzelnen negativen Ladung bedeutet, bevorzugt ein Anion, ausgewählt aus der Gruppe, bestehend aus Fluorid, Chlorid, Bromid, Jodid, Sulfat, Phosphat, Methansulfonat, Nitrat, Maleat, Acetat, Citrat, Fumarat, Tartrat, Oxalat, Succinat, Benzoat und p-Toluolsulfonat,
kombiniert mit einem oder mehreren Betamimetika (**2**), gegebenenfalls in Form der Enantiomeren, Mischungen der Enantiomeren oder in Form der Racemate hiervon, gegebenenfalls in Form der Solvate oder Hydrate, weiterhin enthaltend ein Lösungsmittel, ausgewählt aus Wasser, Ethanol und einer Mischung von Wasser und Ethanol, und gegebenenfalls weiterhin enthaltend einen pharmazeutisch akzeptablen Hilfsstoff, mit der Maßgabe, dass eine Lösung (in mg/100 ml), bestehend aus:
| | |
|---|---|
| einer Verbindung der Formel **1** | 333,3 mg/100 ml, |
| Formoterolfumarat | 333,3 mg/100 ml, |
| Benzalkoniumchlorid | 10,0 mg/100 ml, |
| EDTA | 50,0 mg/100 ml und |
| HCl (1N) | auf pH 3,4 |
ausgeschlossen ist.

2. Treibmittelfreie inhalierbare Lösungen oder Suspensionen nach Anspruch 1, **dadurch gekennzeichnet, dass** die Betamimetika **2** ausgewählt sind aus der Gruppe, bestehend aus Albuterol, Bambuterol, Bitolterol, Broxaterol, Carbuterol, Clenbuterol, Fenoterol, Formoterol, Hexoprenalin, Ibuterol, Isoetharin, Isoprenalin, Levosalbutamol, Mabuterol, Meluadrin, Metaproterenol, Orciprenalin, Pirbuterol, Procaterol, Reproterol, Rimiterol, Ritodrin, Salzneterol, Salmefamol, Soterenot, Sulphonterol, Tiaramid, Terbutalin, Tolubuterol, CHF-1035, HOKU-81, KUL-1248, 3-(4-{6-[2-Hydroxy-2-(4-hydroxy-3-hydroxymethyl-phenyl)-ethytamino]-hexyloxy}-butyl)-benzolsulfonamid, 5-[2-(5,6-Diethyl-indan-2-ylamino)-1-hydroxy-ethyl]-8-hydroxy-1H-chinolin-2-on, 4-Hydroxy-7-[2-{[2-{[3-(2-phenylethoxy)propyl]sulphonyl}ethyl]-amino}ethyl]-2(3H)-benzothiazolon, 1-(2-Fluor-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]etharol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1-1,4-benzoxazin-8-yl]-2-[3-(4-n-butyloxyphenyl)-2-methyl-2-propylamino]ethanol, 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-Hydroxy-8-(1-hydroxy-2-isopropylaminobutyl)-2H-1,4-benzoxazin-3-(4H)-on, 1-(4-Amino-3-chlor-5-trifluormethylphenyl)-2-tert-butylamino)ethanol und 1-(4-Ethoxycarbonylamino-3-cyano-5-fluorophenyl)-2-(tert-butylamino)ethanol, gegebenenfalls in Form der Racemate, der Enantiomeren, der Diastereomeren und gegebenenfalls der pharmakologisch akzeptablen Säureadditionssalze und der Hydrate hiervon.

3. Treibmittelfreie inhalierbare Lösungen oder Suspensionen nach einem der Ansprüche 1 oder 2, **dadurch gekennzeichnet, dass** die Verbindungen **2** in Form der Salze der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure, 1-Hydroxy-2-naphthalincarbonsäure, 4-Phenylzimtsäure, 5-(2,4-Difluorphenyl)salicylsäure oder Maleinsäure oder Mischungen hiervon vorliegen.

4. Treibmittelfreie inhalierbare Lösungen oder Suspensionen nach einem der Ansprüche 1 bis 3, **dadurch gekennzeichnet, dass** die Lösung mit einer organischen oder anorganischen Säure auf einen pH-Wert von 2 bis 7 eingestellt ist.

5. Treibmittelfreie inhalierbare Lösungen oder Suspensionen nach Anspruch 4, **dadurch gekennzeichnet, dass** die Lösung mit einer organischen oder anorganischen Säure auf einen pH-Wert von 2 bis 5 eingestellt ist.

6. Treibmittelfreie inhalierbare Lösungen oder Suspensionen nach einem der Ansprüche 1 bis 3, **dadurch gekennzeichnet, dass** die Gewichtsverhältnisse von **1'** 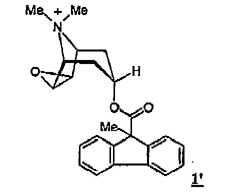 zu **2'** in einem Bereich von etwa 1:5 bis 500:1, bevorzugt 1:10 bis 400:1 liegen.

7. Treibmittelfreie inhalierbare Lösungen oder Suspensionen nach einem der Ansprüche 1 bis 6, **dadurch gekennzeichnet, dass** die Gewichtsverhältnisse von **1'** zu Salmeterol **2'** in einem Bereich von etwa 1:30 bis 400;1, bevorzugt 1:25 bis 200:1 liegen.

8. Treibmittelfreie inhalierbare Lösungen oder Suspensionen nach einem der Ansprüche 1 bis 7, **dadurch gekennzeichnet, dass** die Gewichtsverhältnisse von **1'** 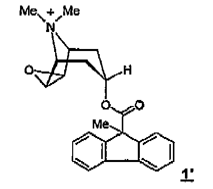 zu Formoterol 2' in einem Bereich von etwa 1:10 bis 300:1, bevorzugt 1:5 bis 200:1 1 liegen.

9. Treibmittelfreie inhalierbare Lösungen oder Suspensionen nach einem der Ansprüche 1 bis 8, **dadurch gekennzeichnet, dass** diese andere Co-Lösungsmittel und/oder Hilfsstoffe enthalten.

10. Treibznittelfreie inhalierbare Lösungen oder Suspensionen nach Anspruch 9, **dadurch gekennzeichnet, dass** diese als Co-Lösungsmittel Bestandteile enthalten, die Hydroxylgruppen oder andere polare Gruppen enthalten, z.B. Alkohol - insbesondere Isopropylalkohol, Glykole - insbesondere Propylenglykol, Polyethylenglykol, Polypropylenglykol, Glycolether, Glycerin, Polyoxyethylenalkohole und Polyoxyethylenfettsäureester.

11. Treibmzttelfreie inhalierbare Lösungen oder Suspensionen nach einem der Ansprüche 9 bis 10, **dadurch gekennzeichnet, dass** diese als Hilfsstoffe oberflächenaktive Mittel, Stabilisatoren, Antioxidantien und/oder Konservierungsmittel, Aromastoffe, pharmakologisch akzeptable Salze und/oder Vitamine enthalten.

12. Treibmittelfreie inhalierbare Lösungen oder Suspensionen nach Anspruch 1, **dadurch** gekennzeichet, dass diese als Komplexienlngsmittel Natriumedetat enthalten,

13. Verwendung von treibmittelfreien inhalierbaren Lösungen oder Suspensionen nach einem der Ansprüche 1 bis 12 zur Herstellung eines Medikaments zur Behandlung von inflammatorischen oder obstruktiven Atemwegsbeschwerden, insbesondere Asthma oder COPD.

## Revendications

1. Solutions ou suspensions inhalables sans propulseur contentant un ou plusieurs sels de formule **1** 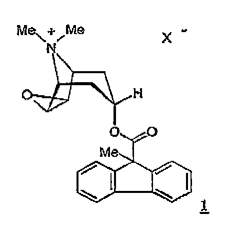 où
X⁻ représente un anion à une seule charge négative, de préférence un anion choisi dans le groupe consistant en fluorure, chlorure, bromure, iodure, sulfate, phosphate, méthanesulfonate, nitrate, maléate, acétate, citrate, fumarate, tartrate, oxalate, succinate, benzoate et p-toluènesulfonate,
combinés avec un ou plusieurs bêtamimétiques (**2**), éventuellement sous forme de leurs énantiomères, de mélanges de leurs énantiomères ou sous forme de leurs racémates, éventuellement sous forme des solvates ou hydrates, contenant en outre un solvant choisi parmi l'eau, l'éthanol et un mélange d'eau et d'éthanol et éventuellement contenant en outre un excipient pharmaceutiquement acceptable, avec la condition qu'une solution (en mg/100 ml) consistant en :
| | |
|---|---|
| un composé de formule **1** | 333,3 mg/100 ml, |
| fumarate de formotérol | 333,3 mg/100 ml, |
| chlorure de benzalkonium | 10,0 mg/100 ml, |
| EDTA | 50,0 mg/100 ml et |
| HCl (1 n) | pour pH 3,4 |
soit exclue.

2. Solutions ou suspensions inhalables sans propulseur selon la revendication 1 **caractérisées en ce que** les bêtamimétiques **2** sont choisis dans le groupe consistant en albutérol, bambutérol, bitoltérol, broxatérol, carbutérol, clenbutérol, fénotérol, formotérol, hexoprénaline, ibutérol, isoétharine, isoprénaline, lévosalbutamol, mabutérol, méluadrine, métaprotérénol, orciprénaline, pirbutérol, procatérol, reprotérol, rimitérol, ritodrine, salmétérol, salméfamol, sotérénot, sulphontérol, tiaramide, terbutaline, tolubutérol, CHF-1035, HOKU-81, KUL-1248, 3-(4-{6-[2-hydroxy-2-(4-hydroxy-3-hydroxyméthyl-phényl)-éthylamino]-hexyloxyl}-butyl)-benzènesulfonamide, 5-[2-(5,6-diéthyl-indan-2-ylamino)-1-hydroxy-éthyl]-8-hydroxy-1*H*-quinolin-2-one, 4-hydroxy-7-[2-{[2-{[3-(2-phényléthoxy)propyl]sulfonyl}éthyl]-amino}éthyl]-2(3H)-benzothiazolone, 1-(2-fluoro-4-hydroxyphényl)-2-[4-(1-benzimidazolyl)-2-méthyl-2-butylamino]éthanol, 1-[3-(4-méthoxybenzyl-amino)-4-hydroxyphényl]-2-[4-(1-benzimidazolyl)-2-méthyl-2-butylamino]éthanol, 1-[2H-5-hydroxy-3-oxo-4H-1,4-benzoxazin-8-yl]-2-[3-(4-N,N-diméthylaminophényl)-2-méthyl-2-propylamino]-éthanol, 1-[2H-5-hydroxy-3-oxo-4H-1,4-benzoxazin-8-yl]-2-[3-(4-méthoxy-phényl)-2-méthyl-2-propylamino]éthanol, 1-[2H-5-hydroxy-3-oxo-4H-1,4-benzoxazin-8-yl]-2-[3-(4-n-butyloxyphényl)-2-méthyl-2-propylamino]éthanol, 1-[2H-5-hydroxy-3-oxo-4H-1,4-benzoxazin-8-yl]-2-{4-[3-(4-méthoxyphényl)-1,2,4-triazol-3-yl]-2-méthyl-2-butylamino}éthanol, 5-hydroxy-8-(1-hydroxy-2-isopropylaminobutyl)-2H-1,4-benzoxazin-3-(4H)-one, 1-(4-amino-3-chloro-5-trifluorométhylphényl)-2-tert.-butylamino)éthanol et 1-(4-éthoxycarbonylamino-3-cyano-5-fluorophényl)-2-(tert.-butylamino)éthanol, éventuellement sous forme de leurs racémates, de leurs énantiomères, de leurs diastéréoisomères et éventuellement de leurs sels d'addition d'acide pharmacologiquement acceptables et de leurs hydrates.

3. Solutions ou suspensions inhalables sans propulseur selon l'une des revendications 1 ou 2 **caractérisées en ce que** les composés **2** sont présents sous forme des sels d'acide chlorhydrique, d'acide bromhydrique, d'acide sulfurique, d'acide phosphorique, d'acide méthanesulfonique, d'acide acétique, d'acide fumarique, d'acide succinique, d'acide lactique, d'acide citrique, d'acide tartrique, d'acide 1-hydroxy-2-naphtalénecarboxylique, d'acide 4-phénylcianamique, d'acide 5-(2,4-difluorophényl)salicylique ou d'acide maléique ou de leurs mélanges.

4. Solutions ou suspensions inhalables sans propulseur selon l'une des revendications 1 à 3 **caractérisées en ce que** la solution est ajustée à un pH de 2 à 7 avec un acide organique ou inorganique.

5. Solutions ou suspensions inhalables sans propulseur selon la revendication 4 **caractérisées en ce que** la solution est ajustée à un pH de 2 à 5 avec un acide organique ou inorganique,

6. Solutions ou suspensions inhalables sans propulseur selon l'une des revendications 1 à 3 **caractérisées en ce que** les rapports massiques de **1'** 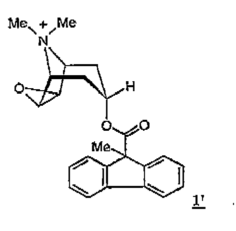 à **2'** sont dans une plage d'environ 1 : 5 à 500 : 1, de préférence de 1 : 10 à 400 : 1.

7. Solutions ou suspensions inhalables sans propulseur selon l'une des revendications 1 à 6 **caractérisées en ce que** les rapports massiques de **1'** au salmétérol **2'** sont dans une plage d'environ 1 : 30 à 400 : 1, de préférence de 1 : 25 à 200 : 1.

8. Solutions ou suspensions inhalables sans propulseur selon l'une des revendications 1 à 7 **caractérisées en ce que** les rapports massiques de **1'** 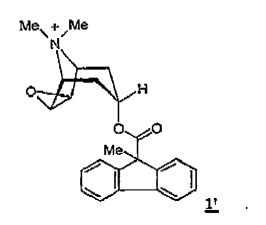 au formotérol **2'** sont dans une plage d'environ 1 : 10 à 300 ; 1, de préférence de 1:5 à 200:1.

9. Solutions ou suspensions inhalables sans propulseur selon l'une des revendications 1 à 8 **caractérisées en ce qu'**elles contiennent d'autres co-solvants et/ou excipients.

10. Solutions ou suspensions inhalables sans propulseur selon la revendication 9 **caractérisées en ce qu'**elles contiennent comme co-solvants des ingrédients qui contiennent des groupes hydroxyle ou d'autres groupes polaires, par exemple des alcools - en particulier l'alcool isopropylique, des glycols - en particulier le propylèneglycol, le polyéthylèneglycol, le polypropylèneglycol, un éther de glycol, le glycérol, les alcools polyoxyéthylénés et les esters d'acide gras polyoxyéthylénés.

11. Solutions ou suspensions inhalables sans propulseur selon l'une des revendications 9 à 10 **caractérisées en ce qu'**elles contiennent comme excipients des tensioactifs, des stabilisants, des antioxydants et/ou des conservateurs, des aromatisants, des sels pharmacologiquement acceptables et/ou des vitamines.

12. Solutions ou suspensions inhalables sans propulseur selon la revendication 1 **caractérisées en ce qu'**elles contiennent comme agent complexant de l'édétate de sodium.

13. Utilisation de solutions ou suspensions inhalables sans propulseur selon l'une des revendications 1 à 12 pour préparer un médicament pour le traitement des affections respiratoires inflammatoires ou obstructives, en particulier l'asthme ou la COPD.
